(19) Europäisches Patentamt
European Patent Office
Office européen des brevets

(11) EP 3 628 375 A1

(12) EUROPEAN PATENT APPLICATION

(43) Date of publication:
**01.04.2020 Bulletin 2020/14**

(21) Application number: **18197210.0**

(22) Date of filing: **27.09.2018**

(51) Int Cl.:
***A61Q 11/00*** *(2006.01)* ***A61K 8/49*** *(2006.01)*
***A61K 8/81*** *(2006.01)* ***A61K 8/86*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Unilever N.V.**
**3013 AL Rotterdam (NL)**

(72) Inventor: **The designation of the inventor has not yet been filed**

(74) Representative: **Tansley, Sally Elizabeth**
**Unilever N.V.**
**Unilever Patent Group**
**Olivier van Noortlaan 120**
**3133 AT Vlaardingen (NL)**

# (54) ORAL CARE COMPOSITION

(57) An oral care composition is disclosed comprising an amphoteric acrylic copolymer, an alkoxylated alcohol based non-ionic surfactant, a pigment having a hue angle, h, in the CIELAB system of from 220 to 320 degrees and a physiologically acceptable carrier.

EP 3 628 375 A1

## Description

### Field of the Invention

The present invention relates to oral care compositions that enhance the white appearance of teeth. More particularly, the present invention relates to oral care compositions comprising pigments. The invention also relates to the use of such compositions for whitening the teeth of an individual.

### Background of the Invention

The enamel layer of the tooth is naturally an opaque white or slightly off-white colour. However, this enamel layer can become stained or discoloured. Many products we consume have a negative impact on our teeth and mouth. Many substances can stain or reduce the whiteness of one's teeth, in particular, certain foods, tobacco products, and fluids such as tea and coffee. These staining and discolouring substances are often able to permeate the enamel layer. This problem occurs gradually over many years, but imparts a noticeable discoloration of the enamel of one's teeth.

Consumers have always had a strong desire for whiter teeth and many individuals are dissatisfied with their current teeth colour. This desire for whiter teeth has given rise to a growing trend in the increased use of tooth whitening products which range from toothpastes to mouthwashes and chewing gums. The whitening effect can be produced by chemically altering or removing the stain and/or changing the visual perception of the color of the teeth.

It is known in the literature that the visual perception of a white substance can be altered through the deposition of an optical brightener, blue pigment or blue dye. Blue Covarine is a phthalocyanine blue pigment present in the toothpaste named white Now® and there have been attempts to enhance the deposition of this pigment on teeth for instant whitening benefits in oral care products.

WO 2016/099544 A1 (Colgate-Palmolive Company) discloses a tooth whitening oral care composition made by combining ingredients comprising flakes of a water soluble whitening film, a dye with a hue angle in the CIELAB system ranging from 200 to 320 degrees and an orally acceptable carrier vehicle.

EP 1 935 395 A1 (Unilever) discloses from 0.01 to 0.3% by weight of a pigment having a hue angle, h, in the CIELAB system of from 220 to 320 degrees, characterized in that the composition further comprises a soluble deposition aid for said pigment.

WO 2012/123241 A2 (Unilever) discloses an oral care composition suitable for delivering a temporary whitening effect to the surface of teeth, the composition comprising: a continuous phase comprising water or polyhydric alcohol or a mixture thereof; a particulate tooth surface whitening agent which is dispersed in the continuous phase, and a deposition aid for the particulate tooth surfaces whitening agent; characterised in that the deposition aid is a poly-(sulphonic acid) polymer. The particulate tooth whitening agent is a phthalocyanine blue pigment.

There is still a need for an oral care composition which delivers improved deposition of pigments on teeth for better tooth whitening benefit.

### Test and definitions

Dentifrice

"Dentifrice" for the purposes of the present invention means a paste, powder, liquid, gum or other preparation for cleaning the teeth or other surfaces in the oral cavity.

Toothpaste

"Toothpaste" for the purpose of the present invention means a paste or gel dentifrice for use with a toothbrush. Especially preferred are toothpastes suitable for cleaning teeth by brushing for about two minutes.

Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

pH

pH is quoted at atmospheric pressure and a temperature of 25°C. When referring to the pH of an oral care

composition, this means the pH measured when 5 parts by weight of the composition is uniformly dispersed and/or dissolved in 20 parts by weight pure water at 25°C. In particular, the pH may be measured by manually mixing 5 g oral care composition with 20 mL water for 30 s, then immediately testing the pH with indicator or a pH meter.

Solubility

"Soluble" and "insoluble" for the purpose of the present invention, refers to the solubility of a source in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

Copolymer

"Copolymer" for the purpose of the present invention, refers to a polymer derived from two or more different species of monomer.

Molecular Weight

"Molecular weight" for the purpose of the present invention, refers to the weight average molecular weight of a given polymer. The weight average molecular weight of polymers is determined by gel permeation chromatography against a polyethylene glycol standard.

Viscosity

Viscosity of a toothpaste is the value taken at room temperature (25°C) with a Brookfield Viscometer, Spindle No.93/94 and at a speed of 5 rpm for 1 minute. Values are quoted in centipoises (cP=mPa.s) unless otherwise specified.

Miscellaneous

Except in the examples, or where otherwise explicitly indicated, all numbers in this description indicating amounts of material or conditions of reaction, physical properties of materials and/or use may optionally be understood as modified by the word "about". All amounts are by weight of the final oral care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

## Summary of the Invention

In a first aspect, the present invention is directed to an oral care composition comprising:

a) an amphoteric acrylic copolymer;
b) an alkoxylated alcohol based non-ionic surfactant;
c) a pigment having a hue angle, h, in the CIELAB system of from 220 to 320 degrees; and
d) a physiologically acceptable carrier.

In a second aspect, the present invention is directed a method of whitening teeth of an individual comprising the step of applying the oral care composition of any embodiment of the first aspect to at least one surface of the teeth of the individual.

All other aspects of the present invention will more readily become apparent upon considering the detailed description and examples which follow.

## Detailed Description of the Invention

The oral care composition of the invention comprises an amphoteric acrylic copolymer. "Amphoteric" as used herein, refers to the polymers have both acid and basic groups and show acidic or basic behaviour depending on the conditions.

The amphoteric acrylic copolymer suitable for use in compositions of the present invention at least comprises constituting units of monomer A and monomer B. Monomer A is a cationic vinyl monomer represented by the formula (I):

$$CH_2=C(R^1)\text{-}CO\text{-}NH\text{-}(CH_2)_mN^+R^3R^4X^- \qquad (I)$$

wherein $R^1$ is a hydrogen atom or a $C_1$-$C_3$ alkyl group, each of $R^2$ to $R^4$ is independently a hydrogen atom or a $C_1$-$C_{24}$ alkyl group, preferably a $C_1$-$C_3$ alkyl group, $X^-$ represents an anion selected from the group of halogens, sulfates, alkylsulfates, hydroxide, phosphate, acetate or formate, and m is an integer from 1 to 12.

$R^1$ is preferably a methyl group. Each of $R^2$ to $R^4$ which are independent from each other, is preferably a methyl group or an ethyl group, more preferably a methyl group. $X^-$ is preferably fluoride, chloride, bromide or iodide anion, more preferably chloride. m is preferably an integer from 1 to 5, more preferably is 3. Most preferably, monomer A is methacrylamidopropyltrimethylammonium chloride.

Monomer B is a $C_3$-$C_8$ unsaturated carboxylic acid represented by the formula (II):

$$CH_2=C(R^5)\text{-}CO(O)Y \qquad (II)$$

wherein $R^5$ is a hydrogen atom or a $C_1$-$C_3$ alkyl group, preferably a hydrogen atom, Y is hydrogen, ammonium, sodium or potassium.

Examples of suitable monomer B include acrylic acid, alkali metal or ammonium salts of acrylic acid. Most preferably monomer B is sodium acrylate.

The molar ratio of monomer A to monomer B is preferably from 1:10 to 10:1, more preferably from 1:6 to 4:1.

Preferably, the amphoteric acrylic copolymer at least comprises constituting units of monomer A and monomer B, wherein monomer A is methacrylamidopropyltrimethylammonium chloride and monomer B is sodium acrylate.

More preferably, the amphoteric acrylic copolymer comprises additional monomers of ethyl acrylate, alkali metal salts of 2-acrylamido-2-methylpropane-sulfonic acid and/or N-isopropylamide, as polymerized monomers.

Preferred amphoteric acrylic copolymer suitable for use in the present invention is methacrylamidopropyltrimethylammonium chloride/sodium acrylate/ethyl acrylate copolymer, methacrylamidopropyltrimethylammonium chloride/ N-isopropylamide/sodium acrylate/sodium 2-acrylamido-2-methylpropane-sulfonate copolymer, or mixtures thereof. Methacrylamidopropyltrimethylammonium chloride/ N-isopropylamide/sodium acrylate/sodium 2-acrylamido-2-methylpropane-sulfonate copolymer is particularly preferred, which is commercially available from BASF under the trade name of Polyquart® Pro A.

The amphoteric acrylic copolymer typically has a molecular weight of from 10,000 to 500,000 g/mol, more preferably from 50,000 to 350,000 g/mol and most preferably from 100,000 to 250,000 g/mol.

Preferably the amphoteric acrylic copolymer is present in an amount of from 0.01 to 5%, more preferably from 0.1 to 5%, even more preferably from 0.2 to 5% and most preferably from 0.3 to 3% by weight, based on total weight of the oral care composition and including all ranges subsumed therein.

The composition of the invention further comprises an alkoxylated alcohol based non-ionic surfactant. Preferably the non-ionic surfactant comprises polyethylene glycol ethers of fatty alcohols. "Fatty alcohol" as used herein, refers to saturated or unsaturated, branched or unbranched $C_8$ to $C_{28}$ alkyl groups with hydroxy substitution. The polyethylene glycol ethers of fatty alcohol are represented by the formula (III):

$$R^6\text{-}[O\text{-}CH_2\text{-}CH_2]_n\text{-}OH \qquad (III)$$

wherein $R^6$ is a saturated or unsaturated, branched or unbranched $C_8$ to $C_{24}$ alkyl groups, preferably a saturated or unsaturated, branched or unbranched $C_{16}$ to $C_{18}$ alkyl groups. n is the degree of ethoxylation from 10 to 100, preferably from 20 to 80, more preferably from 20 to 40. "Degree of ethoxylation" as used herein, refers to the average number of moles of ethylene oxide unit per mole of ethoxylated product.

Examples of suitable alkoxylated alcohol based non-ionic surfactants include the group of surfactants known as Steareth surfactants. Steareth 30 is particularly preferred.

Preferably, the composition comprises from 0.01 to 10%, more preferably from 0.1 to 10%, even more preferably from 0.5 to 8% and most preferably from 0.5 to 5% by weight of the alkoxylated alcohol based non-ionic surfactant, based on total weight of the oral care composition and including all ranges subsumed therein.

It is preferred if the weight ratio of the amphoteric acrylic copolymer to the alkoxylated alcohol based non-ionic surfactant is from 1:20 to 1:1, more preferably from 1:15 to 1:2, even more preferably from 1:10 to 1:4 and most preferably from 1:10 to 1:5.

The pigment according to the invention is a shade/material which is insoluble in the relevant medium, at the relevant temperature. This is in contrast to dyes which are soluble. The term "relevant medium", as used herein, refers to human saliva, the liquid medium in which the composition is used, at the temperature of the oral cavity during brushing of the teeth, i.e. up to 37°C. The relevant medium may also be water and the relevant temperature to be 25°C. The only limitation with respect to the pigment is that the same is suitable for use in the mouth.

The pigment has a hue angle, h, in the CIELAB system of from 220 to 320 degrees, more preferably from 250 to 290 degrees. A detailed description of hue angels may be found on p57 of Colour Chemistry 3rd edition by H. Zollinger published by Wiley-VCH. Preferably the pigment is violet or blue, more preferably the pigment is selected from one or more of those listed in the Colour Index International as pigment blue 1 through to pigment 83 and pigment violet 1 through to pigment violet 56. Other suitable pigments are pigment ultramarine blue and ultramarine violet. While the preferred pigment is blue or violet, the same effect may be achieved through mixing pigments outside of this hue angle range. For example, such a hue angle may also be obtained by mixing a red and green-blue pigment to yield a blue or violet shaded pigment.

It is particularly preferred that the pigment is a blue pigment. Some examples of blue pigments suitable for use in this invention include inorganic blue pigments such as iron blue (C.I. 77510) and ultramarine blue (C.I. 77007). A preferred class of blue pigments suitable for use in the invention are organic blue pigments such as phthalocyanine blue pigments. Phthalocyanines are organometallic compounds containing four symmetrically arranged isoindole rings connected in a 16-membered ring linking with alternate carbon and nitrogen atoms. Most phthalocyanines contains a central, coordinated metal ion such as copper. Copper phthalocyanines have the basis structure:

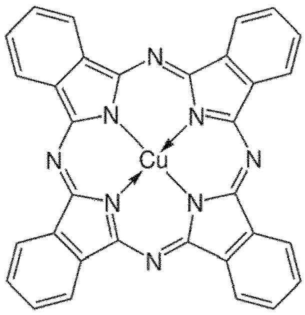

Phthalocyanine blue pigments exhibit more than one crystal modification, which differ in terms of coloristics. Methods have been developed to phase-stabilise the phthalocyanine pigment molecule in order to prevent conversion to a different crystal modification. An example is minor chemical modification of the molecule, for instance partial chlorination. Methods have also been developed to stabilise the phthalocyanine pigment molecule against flocculation during pigment application. An example is admixture of other agents to the molecule, such as surface active additives to the pigment molecule. The following pigments are illustrative phthalocyanine blue pigments preferably included in the composition according to the invention:

| C.I. Generic Name | C.I. Constitution Number | Crystal modification; Type | Number of halogen atoms |
|---|---|---|---|
| Pigment Blue 15 | 74160 | alpha | -- |
| Pigment Blue 15:1 | 74160 | alpha; phase stabilised | 0.5-1 Cl |
| Pigment Blue 15:2 | 74160 | alpha; phase & flocculation stabilised | 0.5-1 Cl |
| Pigment Blue 15:3 | 74160 | beta | -- |
| Pigment Blue 15:4 | 74160 | beta; flocculation stabilised | -- |
| Pigment Blue 15:6 | 74160 | epsilon | -- |
| Pigment Blue 16 | 74100 | gamma; metal-free | -- |

It is especially preferred that the pigment is phthalocyanine blue pigment selected from alpha copper phthalocyanines Pigment Blue 15, Pigment Blue 15:1, Pigment 15:2 and mixtures thereof. Most preferably the pigment is

Pigment Blue 15:1. A commercially available example is Cosmenyl Blue A4R from Clariant.

The pigment suitable for use in this invention may also be mixtures of any of the above described materials.

The oral care composition of the present invention typically comprises from 0.001 to 1% by weight of the pigment, more preferably from 0.01 to 0.5%, and most preferably from 0.02 to 0.2%, based on total weight of the oral care composition and including all ranges subsumed therein.

The oral care composition may also comprise other surfactants in addition to the alkoxylated alcohol based non-ionic surfactant. If other surfactants are present, it is preferred if the alkoxylated alcohol based non-ionic surfactant is at least 75% by weight of the surfactant in the composition, more preferably from 80 to 100% and most preferably from 95 to 100% based on total weight of the surfactant in the composition. Suitable surfactants include anionic surfactants, such as the sodium, magnesium, ammonium or ethanolamine salts of $C_8$ to $C_{18}$ alkyl sulphates (for example sodium lauryl sulphate), ethoxylated alkyl sulphate (such as sodium lauryl ether sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include amphoteric surfactants, $C_8$ to $C_{19}$ alkyl amine oxides, $C_8$ to $C_{19}$ alkyl glycinates, $C_8$ to $C_{19}$ alkyl carboxyglycinates, $C_8$ to $C_{19}$ alkyl amphoacetates, $C_8$ to $C_{19}$ alkyl amphopropionates, $C_8$ to $C_{19}$ alkylamphoglycinates, $C_8$ to $C_{19}$ acyl taurates and acyl glutamates. Mixtures of any of the above described materials may also be used. Although other surfactants may be present, it is preferred if they are limited to levels below 0.5% by weight of the oral care composition, preferably less than 0.1% by weight of the oral care composition.

Preferably, the oral care composition has a pH from 4.0 to 10.0, more preferably from 5.0 to 9.0, and most preferably from 5.5 to 8.0.

The composition of the present invention is an oral care composition and comprises a physiologically acceptable carrier. The carrier preferably comprises at least thickener, humectant or a combination thereof.

Thickener may be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, xanthan gum and/or sodium carboxymethyl cellulose and/or a Carbomer is/are preferred. When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

In another especially preferred embodiment, the thickener is xanthan gum.

In another especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

Thickener typically makes up from 0.01 to about 10%, more preferably from 0.1 to 9%, and most preferably, from 0.1 to 5% by weight of the oral care composition, based on total weight of the composition and including all ranges subsumed therein.

The oral care composition of the invention is preferably a toothpastes or gel. When the oral care composition is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 30 to 70% by weight of the composition, based on total weight of the composition and including all ranges subsumed therein.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, pH-adjusting agents,

sweetening agents, particulate abrasive materials, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of whitening the teeth of an individual comprising applying the composition to at least one surface of the teeth of the individual. The oral care composition of this invention may additionally or alternatively be for use as a medicament and/or used in the manufacture of a medicament for providing an oral care benefit as described herein, such as for whitening the teeth of an individual. Alternatively and preferably, the use is non-therapeutic.

Typically, the composition will be packaged. In toothpaste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day.

The following examples are provided to facilitate an understanding of the present invention. The examples are not provided to limit the scope of the claims.

## Examples

### Example 1

This example demonstrated the deposition of pigments by using a combination of polymers and surfactants. All ingredients are expressed by weight percent of the total formulation and as level of active ingredient.

**TABLE 1**

| Ingredients | Samples | | | | | | | | | |
|---|---|---|---|---|---|---|---|---|---|---|
| | 1 | 2 | 3 | 4 | 5 | 6 | 7 | 8 | 9 | 10 |
| PQ-7[a] | 1.00 | 1.00 | -- | -- | -- | -- | -- | -- | -- | -- |
| Jaguar BB18[b] | -- | -- | 1.00 | 1.00 | -- | -- | -- | -- | -- | -- |
| Polyquart Pro A[c] | -- | -- | -- | -- | 1.00 | 1.00 | 1.00 | -- | -- | -- |
| Gantrez S97[d] | -- | -- | -- | -- | -- | -- | -- | 1.00 | 1.00 | 1.00 |
| Steareth 30 | 1.80 | -- | 1.80 | -- | 1.80 | -- | -- | 1.80 | -- | -- |
| Cocamidopropyl betaine | -- | 1.80 | -- | 1.80 | -- | 1.80 | -- | -- | 1.80 | -- |
| Sodium lauryl sulfate | -- | -- | -- | -- | -- | -- | 1.8 | -- | -- | 1.80 |
| Blue Covarine[e] (37.8%) | 0.075 | 0.075 | 0.075 | 0.075 | 0.075 | 0.075 | 0.075 | 0.075 | 0.075 | 0.075 |
| Water | To 100 | To 100 | To 100 | To 100 | To 100 | To 100 | To 100 | To 100 | To 100 | To 100 |

a. Commercial polyquaternium-7 under the trade name Merquat 550 from Lubrizol
b. Commercial guar hydroxypropyltrimmonium chloride under the trade name Jaguar BB18 from Solvay Novecare
c. Commercial acrylamidopropyltrimethylammonium chloride/sodium acrylate/N-isopropylamide/2-acrylamido-2-methylpropane-sulfonic acid copolymer under the trade name Polyquart Pro A from BASF
d. Commercial copolymer of methyl vinyl ether and maleic anhydride under the trade name Gantrez S97 from Ashland
e. Dispersion of C.I. 74160 (pigment blue 15:1) in water/glycerol under the trade name Cosmenyl Blue A4R from Clariant.

*Methods*

To evaluate the deposition of pigments, the following in vitro test was performed. The colour difference expressed as the CIELAB delta E (ΔE) value was calculated based on L*, a*, b* values which were measured using DigiEye (VeriVide, England) with the following formula:

$$\Delta E = \sqrt{\Delta L^2 + \Delta a^2 + \Delta b^2}$$

Four hydroxyapatite (HAP) discs were used for each testing group. They were kept in deionized (DI) water for 1 hour before brushing. The baseline L*, a*, b* of the HAP discs was measured by DigiEye (VeriVide, England).

The test sample was shaken on minishaker for 30 seconds before use. The HAP discs were brushed with the slurry for one minute on a WIRA® brushing machine equipped with toothbrushes. The load of the tooth brushing was 175 g and the automatic brushing operated at a speed of 150 rpm. Thereafter the HAP discs were rinsed three times, and each time with 20 mL DI water for 30 seconds by the brushing machine. After this step, the L*, a*, b* values of each HAP disc were re-measured based on which the delta E from baseline were calculated and statistically analyzed.

*Results*

The results are summarized in Table 2 (error represents standard error for duplicate measurements). The higher the delta E value the more deposition of the pigment.

**TABLE 2**

| | Samples | | | | | | | | | |
|---|---|---|---|---|---|---|---|---|---|---|
| | **1** | **2** | **3** | **4** | **5** | **6** | **7** | **8** | **9** | **10** |
| ΔE | 2.02±0.13 | 2.85±0.32 | 1.07±0.18 | 1.89±0.19 | 5.45±0.38 | 2.95±0.16 | 2.14±0.59 | 3.78±0.37 | 1.24±0.10 | 2.33±0.65 |

Sample 5 comprising a combination of Pro A and steareth 30 showed significantly higher ΔE values ($p<0.05$) compared to other samples, which indicated much better deposition of pigments.

Example 2

This example demonstrated the concentration of polymers or surfactants can affect the deposition of pigments. All ingredients are expressed by weight percent of the total formulation, and as level of active ingredient.

**TABLE 3**

| **Ingredients** | **Samples** | | | | | |
|---|---|---|---|---|---|---|
| | **11** | **12** | **13** | **14** | **15** | **16** |
| Polyquart Pro A[c] | 0.10 | 0.55 | 1.00 | 0.55 | 0.55 | 0.55 |
| Steareth 30 | 2.00 | 2.00 | 2.00 | 1.00 | 3.00 | 5.00 |
| Blue Covarine[e] (37.8%) | 0.075 | 0.075 | 0.075 | 0.075 | 0.075 | 0.075 |
| Water | To 100 | To 100 | To 100 | To 100 | To 100 | To 100 |

*Methods*

The same protocol was used to evaluate the deposition of pigments as described in Example 1.

*Results*

The results are summarized in Table 4 (error represents standard error for duplicate measurements).

**TABLE 4**

| | Samples | | | | | |
|---|---|---|---|---|---|---|
| | **11** | **12** | **13** | **14** | **15** | **16** |
| ΔE | 2.07±0.43 | 10.46±0.37 | 16.68±1.46 | 3.62±0.19 | 18.29±0.42 | 15.34±0.48 |

Sample 13 comprising a higher concentration of Pro A had significantly higher ΔE ($p<0.05$) values than Samples 11 and 12. The results also showed that Sample 15 significantly higher ΔE ($p<0.01$) values compared to Samples 12, 14 and 16.

Example 3

This example demonstrated the effect of surfactants on the deposition of pigments. All ingredients are expressed by weight percent of the total formulation, and as level of active ingredient.

**TABLE 5**

| **Ingredients** | **Samples** | | |
|---|---|---|---|
| | **17** | **18** | **19** |
| Polyquart Pro A[c] | 1.00 | 1.00 | 1.00 |
| Steareth 30 | 1.80 | -- | -- |
| Tween 80 | -- | 1.80 | -- |
| Triton X-100 | -- | -- | 1.80 |
| Blue Covarine[e] (37.8%) | 0.075 | 0.075 | 0.075 |
| Water | To 100 | To 100 | To 100 |

*Methods*

The same protocol was used to evaluate the deposition of pigments as described in Example 1.

*Results*

The results are summarized in Table 6 (error represents standard error for duplicate measurements).

**TABLE 6**

| | **Samples** | | |
|---|---|---|---|
| | **17** | **18** | **19** |
| ΔE | 14.69 ± 0.29 | 2.11 ± 0.18 | 7.38 ± 0.12 |

The results showed that Sample 17 comprising Steareth 30 had significantly higher ΔE ($p<0.01$) values than Samples 18 and 19 comprising other surfactants.

## Claims

**1.** An oral care composition comprising:

a) an amphoteric acrylic copolymer;
b) an alkoxylated alcohol based non-ionic surfactant;
c) a pigment having a hue angle, h, in the CIELAB system of from 220 to 320 degrees; and
d) a physiologically acceptable carrier.

2. The oral care composition according to claim 1, wherein the amphoteric acrylic copolymer at least comprises constituting units of monomer A and monomer B, and wherein monomer A is methacrylamidopropyltrimethylammonium chloride and monomer B is sodium acrylate

3. The oral care composition according to claim 1 or claim 2, wherein the amphoteric acrylic copolymer is acrylamidopropyltrimethylammonium chloride/sodium acrylate/ethyl acrylate copolymer, methacrylamidopropyltrimethylammonium chloride/ N-isopropylamide/sodium acrylate/sodium 2-acrylamido-2-methylpropanesulfonate copolymer or mixtures thereof, preferably methacrylamidopropyltrimethylammonium chloride/ N-isopropylamide/sodium acrylate/sodium 2-acrylamido-2-methylpropane-sulfonate copolymer.

4. The oral care composition according to any of the preceding claims, wherein the amphoteric acrylic copolymer has a molecular weight of from 10,000 to 500,000 g/mol, preferably from 50,000 to 350,000 g/mol.

5. The oral care composition according to any of the preceding claims, wherein the composition comprises from 0.01 to 5% by weight of the amphoteric acrylic copolymer, preferably from 0.1 to 5%.

6. The oral care composition according to any of the preceding claims, wherein alkoxylated alcohol based non-ionic surfactant comprises polyethylene glycol ethers of fatty alcohols.

7. The oral care composition according to claim 6, wherein the polyethylene glycol ethers of fatty alcohols have a degree of ethoxylation from 10 to 100, preferably from 20 to 80.

8. The oral care composition according to any of the preceding claims, wherein the alkoxylated alcohol based non-ionic surfactant is steareth 30.

9. The oral care composition according to any of the preceding claims, wherein the composition comprises from 0.01 to 10% by weight of the alkoxylated alcohol based non-ionic surfactant, preferably from 0.1 to 10%.

10. The oral care composition according to any of the preceding claims, wherein the weight ratio of the amphoteric acrylic copolymer to the alkoxylated alcohol based non-ionic surfactant is from 1:20 to 1:1, preferably from 1:15 to 1:2.

11. The oral care composition according to any of the preceding claims, wherein the pigment has a hue angel, h, in the CIELAB system of from 250 to 290.

12. The oral care composition according to claim 11, wherein the pigment is a blue pigment, preferably a phthalocyanine blue pigment.

13. The oral care composition according to claim 12, wherein the phthalocyanine blue pigment is selected from alpha copper phthalocyanines Pigment Blue 15, Pigment Blue 15:1, Pigment Blue 15:2 and mixtures thereof, preferably Pigment Blue 15:1.

14. The oral care composition according to any of the preceding claims, wherein the composition comprises from 0.001 to 1% by weight of the pigment, preferably from 0.01 to 0.5%.

15. A method of whitening teeth of an individual comprising the steps of applying the composition according to any of the preceding claims to at least one surface of the teeth of an individual.

Europäisches Patentamt
European Patent Office
Office européen des brevets

# EUROPEAN SEARCH REPORT

Application Number
EP 18 19 7210

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2012/123241 A2 (UNILEVER PLC [GB]; UNILEVER NV [NL] ET AL.) 20 September 2012 (2012-09-20) * example 1 * | 1-15 | INV. A61Q11/00 A61K8/49 A61K8/81 A61K8/86 |
| A | WO 2018/088995 A1 (COLGATE PALMOLIVE CO [US]) 17 May 2018 (2018-05-17) * paragraph [0075]; claims 1,14 * | 1-15 | |
| A | WO 2015/095709 A1 (COLGATE PALMOLIVE CO [US]) 25 June 2015 (2015-06-25) * paragraphs [0098], [0127] * | 1-15 | |
| A | WO 2011/053877 A2 (DISCUS DENTAL LLC [US]; MONTGOMERY R ERIC [US]) 5 May 2011 (2011-05-05) * claims 1,7; examples * | 1-15 | |

TECHNICAL FIELDS SEARCHED (IPC)

A61K
A61Q

The present search report has been drawn up for all claims

| Place of search | Date of completion of the search | Examiner |
|---|---|---|
| Munich | 26 March 2019 | Werner, Stefan |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 19 7210

This annex lists the patent family members relating to the patent documents cited in the above-mentioned European search report. The members are as contained in the European Patent Office EDP file on
The European Patent Office is in no way liable for these particulars which are merely given for the purpose of information.

26-03-2019

| Patent document cited in search report | Publication date | Patent family member(s) | Publication date |
|---|---|---|---|
| WO 2012123241 A2 | 20-09-2012 | BR 112013021953 A2 | 12-06-2018 |
| | | CN 103402584 A | 20-11-2013 |
| | | EP 2683447 A2 | 15-01-2014 |
| | | WO 2012123241 A2 | 20-09-2012 |
| WO 2018088995 A1 | 17-05-2018 | NONE | |
| WO 2015095709 A1 | 25-06-2015 | AU 2014369061 A1 | 16-06-2016 |
| | | CA 2927282 A1 | 25-06-2015 |
| | | CL 2016001549 A1 | 17-02-2017 |
| | | MX 354968 B | 27-03-2018 |
| | | PH 12016501080 A1 | 11-07-2016 |
| | | RU 2016124326 A | 23-01-2018 |
| | | US 2016331653 A1 | 17-11-2016 |
| | | US 2019021963 A1 | 24-01-2019 |
| | | WO 2015095709 A1 | 25-06-2015 |
| WO 2011053877 A2 | 05-05-2011 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016099544 A1
- EP 1935395 A1
- WO 2012123241 A2


### Non-patent literature cited in the description


- **H. ZOLLINGER.** Colour Chemistry. Wiley-VCH, 57